# EUROPEAN PATENT APPLICATION

(11) **EP 0 860 158 A1**
(43) Date of publication of application: **26.08.1998**
(21) Application number: 96120567.1
(22) Date of filing: 20.12.1996
(51) Int. Cl.: A61F 13/15

(54) **Process for manufacturing individual layered structures comprising particulate material**

(71) Applicant: THE PROCTER & GAMBLE COMPANY, Cincinnati, Ohio 45202 (US)
(72) Inventor: Gagliardi, Ivano, 65123 Pescara (IT); Carlucci, Giovanni, 66100 Chieti (IT); Febo, Pietro, 65126 Pescara (IT)
(74) Representative: Hirsch, Uwe Thomas

(57) **Abstract**

A process for manufacturing individual layered structures comprising at least two containing layers comprising the steps of:
a) providing a continuous web-like layered structure having designated cutting lines and comprising a particulate material between containing layers;
b) cutting the continuous web-like layered structure along the designated cutting lines to provide individual layered structures having end surfaces between cuts;
c) spacing out the individual layered structures from one another so as to provide spaces between the end surfaces;
d) applying an adhesive composition to at least part of the end surfaces, so that the adhesive composition provides joining in the at least part of the end surfaces between the containing layers.

## Description

### FIELD OF THE INVENTION

The present invention relates to a process for manufacturing individual layered structures comprising a particulate material, which process prevents the loss or spillage of the particulate material from the structures. Preferably, the individual layered structures comprise absorbent fibrous layers and can be used as absorbent elements in disposable absorbent articles such as absorbent articles for incontinent adults, babies' nappies, sanitary towels, dressings and the like.

### BACKGROUND OF THE INVENTION

Disposable absorbent articles are well known and all have absorbent elements for absorbing and retaining body fluids; an absorbent element must be able to acquire liquid rapidly and to distribute it internally so as to prevent leakage and must also have a good capacity to retain the fluids when subjected to the normal pressures of use.

Absorbent elements made mainly of hydrophilic fibrous material such as, for example, pads of cellulose fibres, layers of wadding, or the like generally have satisfactory characteristics as regards their liquid-absorption rate and can distribute the liquid effectively within them but are very ineffective from the point of view of retention when subjected to the normal pressures of use.

The use of absorbent gelling materials in combination with hydrophilic fibres in order to increase the absorption and retention capacities of the absorbent elements is known.

Absorbent gelling materials, commonly known as superabsorbents, are polymers which can swell up and absorb large quantities of liquid, particularly water, or also, to a lesser extent, body fluids.

They also have the particular property that they retain the fluids even under moderate pressure; owing to this characteristic, their use in absorbent elements for disposable absorbent articles has been proposed for some time.

With the use of absorbent gelling materials, it is possible to produce absorbent elements which contain less hydrophilic fibres for a given absorption capacity and which consequently have smaller dimensions, particularly thicknesses, than conventional absorbent elements made of fibres alone.

Absorbent gelling materials are commonly incorporated in particle form within fibrous structures. Structures have been formed in which the fibres and the particles of absorbent gelling material are disposed in separate, generally very thin, superposed layers or, alternatively, the particles can also be mixed with fibres in one of the fibrous layers.

Many particular forms of layered, absorbent structures of this type, in which the fibrous material is represented by one of more layers of wadding, absorbent paper or non-woven fabric, and in which the particles of absorbent gelling material are incorporated in the structure in various ways, are known in the art.

Different types of particulate materials, other than absorbent gelling material particles, can also be incorporated in layered absorbent structures, such as for example odour control materials in particle or powder form.

Usually a continuous, web-like layered absorbent structure is manufactured, which is subsequently cut in smaller pieces to provide the individual layered absorbent structures to be used as absorbent elements in disposable absorbent articles.

Layered absorbent structures incorporating particulate material can be formed directly on the production line for the absorbent articles in which they are to be incorporated, or, alternatively, they can be produced independently as semi-finished products that are sold and stored separately in form of a continuous web-like structure, e.g. wound in a roll, or otherwise held in a container, and are then fed to the production line.

A common problem with layered absorbent structures incorporating particulate material consists in the effective containment of the particles within the structure in a stable manner, e.g. avoiding that the particles move within the structure and become locally concentrated. The particulate material can also escape from the edges of the layered structure, particularly along the edges where the individual structures are cut from the continuous web-like structure, therefore creating a problem both in the production line and in the final product.

A known solution for the formation of a layered absorbent structure provides for the use of an adhesive, e.g. of the hot melting type, applied to the entire surface of one of the fibrous layers with the dual purpose of bonding the two fibrous layers together and simultaneously fixing the particles of e.g. absorbent gelling material and/or odour control material between them.

The use of an adhesive may, however, affect the characteristics both of the fibrous layer to which the adhesive is applied, and of the particulate material which comes into contact with the adhesive.

In general, therefore, it is necessary not to use an excessive quantity of adhesive and consequently the possibility of the loss of particulate material from the edges of the layered structure cannot be completely eliminated.

Different solutions to the problem of the loss of particulate material along the longitudinal edges of a continuous, web-like layered absorbent structure are known, by longitudinal edges being meant the edges of the layered structure that are parallel to the direction of formation of the structure itself.

For example, the continuous, web-like, layered absorbent structure can be completely surrounded with a layer of wadding, or, alternatively, the structure can be provided by means of a single layer of fibrous material on which the adhesive and the particulate material are distributed only on a central longitudinal strip and subsequently the two side portions are folded so that they partially overlap approximately along the longitudinal axis, and are joined e.g. by means of adhesive.

In International Patent Applications WO 94/01069 and WO 95/17868 by Procter & Gamble Company continuous, web-like, thin, layered absorbent structures containing absorbent gelling material for use as absorbent elements in disposable absorbent articles are described. Such layered absorbent structures are formed by at least two fibrous layers comprising between them a layer of particles of absorbent gelling material, the two fibrous layers being joined together by particles of thermoplastic, polymeric, organic material in finely divided form distributed and mixed with the absorbent gelling material, and by two lines of adhesive disposed along the longitudinal edges of the structure.

While the above described layered structures do not have the problem of loss of particulate material along the longitudinal edges, they still have a problem of loss or spillage of particles when they are cut transversely relative to the longitudinal direction in the production line in order to form the individual layered absorbent structures that are to be incorporated as absorbent elements in the absorbent articles. Not only in fact the transverse cuts open the layered structure, therefore exposing the particulate material comprised therebetween, but the cutting action itself tends to break the particles with formation of powders and, which is more likely to occur in the layered absorbent structures described in the two above mentioned international applications, it can also break the bonding points created by the melted polymeric material between the particles and the fibrous layers. The problem of the loss of particulate material along the cut edges is therefore enhanced by these effects.

U.S. Patents 4,715,918 by Kimberly-Clark Corporation and 4,646,510 by Acumeter Lab. Inc., and European Patent EP-B-22792 by Beghin Say SA describe layered structures with particles embedded within two fibrous layers and confined in so called pockets or pouches which are created by the two fibrous layers joined by means e.g. of adhesive or fibre entanglement. The particles are to be selectively deposited on the substrate only on predetermined zones in order to form the pockets or pouches. These methods are capable of achieving a structure sealed both in longitudinal and in transverse direction, but are rather complex and are not suitable to produce pre-formed laminated structures that are to be subsequently fed to a production line and cut at predetermined intervals to form the individual layered absorbent structures.

It is therefore an object of the present invention to provide a process for manufacturing individual layered structures comprising a particulate material, which process prevents the loss or spillage of particulate material from the structures incorporating such material, particularly during the cutting step by which the individual layered structures are cut from a larger layered structure, e.g. a continuous, web-like layered structure.

It is a further object of the present invention to provide such a process that is applicable both to layered structures that are formed directly on the production line for the articles in which they are to be incorporated, and to continuous, web-like, layered structures that are produced as semi-finished intermediate products and are intended to be later fed to the production line.

### SUMMARY OF THE INVENTION

The present invention relates to a process for manufacturing individual layered structures comprising the steps of:
a) providing a continuous web-like layered structure having designated cutting lines and comprising a particulate material between containing layers;
b) cutting the continuous web-like layered structure along the designated cutting lines to provide individual layered structures having end surfaces between cuts;
c) spacing out the individual layered structures from one another so as to provide spaces between the end surfaces;
d) applying an adhesive composition to at least the end surfaces along at least part of the cutting lines, so that the adhesive composition provides the containing layers in at least part of the cutting lines with a joining means.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of a continuous web-like layered structure that can be employed to form individual layered structures according to the process of the present invention;
FIG. 2 is a perspective view of a continuous web-like layered structure of the type illustrated in FIG. 1, wound in a roll;
FIG. 3 is a perspective view of an individual layered structure being made from a continuous, web-like layered structure in accordance with the present invention;
FIG. 4 is is a cross-sectional view of a portion of the individual layered structure shown in FIG. 1 comprising an end surface, as taken along a section line corresponding to the longitudinal centreline A-A;
FIG. 5 is a schematic flow diagram of a process for making individual layered absorbent structures and for incorporating them in absorbent articles according to the present invention.

### DETAILED DESCRIPTION OF THE INVENTION

The invention relates to a process for manufacturing individual layered structures from a larger web-like layered structure; the larger web-like layered structure is a continuous structure and comprises a particulate material between containing layers. Individual layered structures are cut from the larger web-like layered structure and are joined along the cut so as to prevent loss or spillage of the particulate material from the edges thereof. In a preferred embodiment, the individual layered structures comprise fibrous layers that are preferably liquid absorbent. The individual layered structures of the present invention will be described herein in relation to their use as absorbent elements in disposable absorbent articles, but they can be used for different purposes, for example as an absorbent structure for a cleaning article.

Disposable absorbent articles are intended to be articles that are worn by the user in direct contact with the body; their purpose is to absorb body fluids and they are then thrown away after a single use.

The individual layered structures of the present invention can constitute integrally the absorbent element of a disposable absorbent article, or they can be comprised therein as part of the absorbent element, or in any case they can constitute an element of a disposable absorbent article.

Disposable absorbent articles, such as for example sanitary napkins, pantiliners, incontinent pads, or diapers, typically comprise a fluid pervious topsheet, a fluid impervious backsheet, that can optionally be water vapour and/or gas pervious, and an absorbent element comprised therebetween.

The term "layered", as used herein, indicates any structure in which layers containing particulate material are recognizable from layers that substantially do not comprise particulate material. This comprises structures in which the containing layers and the particulate material are disposed in separate, superposed layers or, alternatively, structures in which the particulate material is embedded in a given position within the thickness of a single layer, e.g. a fibrous layer, for example in correspondence of the centre of the layer, in order to prevent particulate material from escaping from one or both major flat surfaces of the layered structure.

The term "cutting line", as used herein, indicates a path where the continuous web-like layered structure has to be cut in order to provide individual layered structures. A cutting line can comprise a closed perimeter or, alternatively or in combination, cutting lines that are separate from each other.

The structure can also comprise non fibrous containing layers, for example a polymeric film layer.

The process of the present invention for manufacturing individual layered absorbent structures will be described herein with reference to a continuous, web-like, layered absorbent structure which is similar to the thin layered absorbent structures described in the two above cited international applications WO 94/1069 and WO 95/17868.

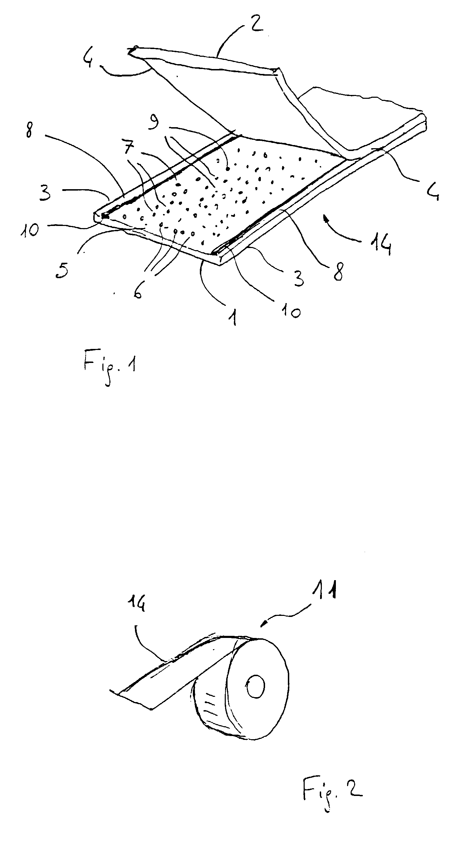

Figure 1 shows a continuous, web-like, layered absorbent structure 14 with one of the layers partially raised to show its construction more clearly.

In Figure 1 it is possible to distinguish a first fibrous layer 1 and a second fibrous layer 2 in the form of two continuous strips of the same width, which are superposed so that their respective longitudinal edges 3 and 4 coincide; the fibrous layers constitute the containing layers and can be made of various materials such as, for example, paper, wadding, or non-woven fabric; they are preferably made of dry-formed layers, generally referred to as "air laid" layers, of short cellulose fibres having a basic weight of between 20 g/m² and 150 g/m².

Alternatively, the two fibrous layers can be made of different materials, for example the second fibrous layer 2 may consist of a dry-formed mixture of cellulose fibres and bicomponent polyethylene/polypropylene fibres, such as, for example, those sold by Danaklon a/s of Varde, Denmark, as AL-Thermal B and AL-Thermal C.

Between the two containing fibrous layers 1 and 2 there is an intermediate layer 5 of particulate material made of a mixture of particles of absorbent gelling material 6, particles of odour control material 9 and a thermoplastic, polymeric, organic material in finely divided form, preferably in form of particles 7; the width of the said intermediate layer 5 is less than that of the outer two fibrous layers 1 and 2 which extend beyond the intermediate layer 5 laterally forming two longitudinal edge portions 8 at their respective longitudinal edges 3 and 4.

The longitudinal direction typically corresponds to the direction of formation of the continuous, web-like layered absorbent structure 14.

The two outer fibrous layers 1 and 2 are bonded together in the central region in which the intermediate layer 5 is present by the application of heat and moderate pressure to melt the particles 7 of thermoplastic, polymeric, organic material present in the intermediate layer 5, mixed with the particles 6 of absorbent gelling material, and 9 of odour control material.

The bond between the fibrous layers 1 and 2 is generated by the melting of the individual particles 7 of thermoplastic polymeric, organic material; as it melts, the polymeric material forms "bridges" connecting directly the fibrous layers 1 and 2 and/or also comprising particles 6 of absorbent gelling material, and particles 9 of odour control material.

It has to be noted that in the finished product the term "particulate material" identifies only the particles 6 of absorbent gelling material and 9 of odour control material, since the thermoplastic polymeric material after the melting step is no more in form of particles 7.

The overall surface area of the bond points represents a small fraction of the surface area of the fibrous layers 1 and 2 and of the particles of absorbent gelling material and odour control material, the characteristics of which thus remain almost unchanged.

Two continuous lines 10 of adhesive are also applied to the two sides of the intermediate layer 5 on the longitudinal edge regions 8 of the two outer fibrous layers 1 and 2 so as to prevent the particulate material, i.e. particles of absorbent gelling material 6 and of odour control material 9 from escaping from the longitudinal edges of the layered structure, which correspond to the superposed edges 3 and 4 of the two fibrous layers, and also to reinforce the connection between the fibrous layers themselves.

The containing layers and the particulate material can be alternatively bonded together by different means, other than the thermoplastic polymeric material in finely divided form, e.g. in form of particles 7, and the continuous lines of adhesive 10 described herein; for example, a sprayed layer of adhesive can replace at least the thermoplastic polymeric material in finely divided form.

Any other means for bonding the containing layers together along the respective longitudinal edges can also be used in alternate embodiments of the present invention instead of the continuous lines of adhesive, e.g. fusion bonding.

The absorbent gelling material, which is preferably distributed in the form of particles 6, may be made of inorganic or organic substances such as cross-linked polymers, all known from the prior art. The odour control material can be any suitable odour control material known in the art, for example it can be constituted by particles of zeolite and silica.

The average dimensions of the particles 6 and 9, given as a weighted average of the smallest dimensions of the individual particles, can be between 50 microns and 1500 microns, preferably between 100 microns and 800 microns.

The quantity of the absorbent gelling material 6 in the intermediate layer 5 can range from 20 g/m², up to 600 g/m². The quantity of odour control material 9 can be between 40 g/m² and 200 g/m².

The thermoplastic, polymeric, organic material in finely divided form, e.g. in form of particles 7 has the purpose of bonding the two fibrous layers 1 and 2 together by melting and forming discrete, spaced-apart bond points between the fibres of the two layers. The thermoplastic, polymeric, organic material can also be used in other finely divided forms, e.g. in form of fibres.

As explained above, the bridges which form these bond points can involve particles of absorbent gelling material and odour control material.

The quantity of thermoplastic, polymeric, organic material in finely divided form distributed and mixed with the absorbent gelling material can be between 5 g/m² and 180 g/m².

The thermoplastic, polymeric, organic material in finely divided form can preferably be melted at a temperature such as not to interfere with the characteristics of the other components of the layered structure, i.e. the fibrous layers and the particulate material, namely the absorbent gelling material and the odour control material. Therefore, the thermoplastic polymeric organic material must have fluidity characteristics such as to enable the necessary bonds to be formed rapidly.

These preferred characteristics can be achieved by a thermoplastic, polymeric, organic material in finely divided form having a melt flow index (M.F.I.), evaluated by the ASTM method D 1238-85 under conditions 190/2.16, of at least 25 g/10 min, preferably at least 40 g/10 min, and even more preferably at least 60 g/10 min.

If the layers 1 and 2 are made of a dry-formed short cellulose fibre material, it is preferable to use a thermoplastic, polymeric, organic material composed of particles of high-density polyethylene with maximum dimensions of about 400 microns, characterized by a melt flow index of about 50 g/10 min, of which the quantity distributed is between 12 g/m² and 90 g/m²

The continuous, web-like, layered absorbent structure 14 may also be formed by two different fibrous layers or may comprise more than two fibrous layers, and consequently more than one intermediate layer formed by the mixture of particulate material, e.g. particles of absorbent gelling material and of odour control material, and particles of thermoplastic, polymeric, organic material.

Of course, provided that a particulate material is actually comprised between containing layers, any of the absorbent gelling material, odour control material, or thermoplastic, polymeric, organic material in finely divided form comprised in a preferred layered structure made according to the process of the present invention can be in a form which is different from the particulate form. For example, the absorbent gelling material can be in fibrous form, while the odour control material can be comprised as a solution sprayed onto a substrate; the thermoplastic, polymeric, organic material in finely divided form can also be in fibrous form, as already mentioned above.

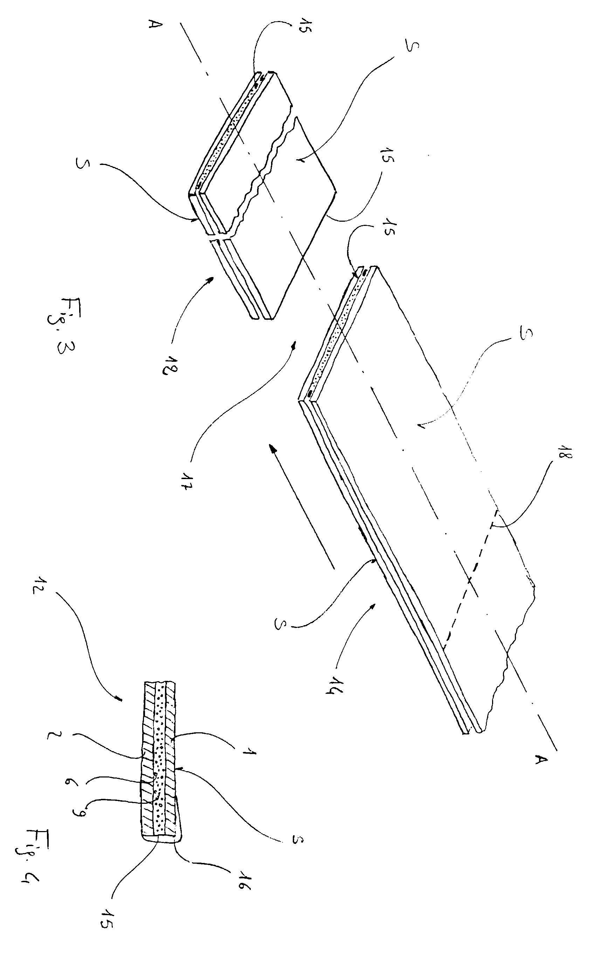

The continuous lines 10 of adhesive disposed between the fibrous layers on the respective longitudinal edge portions prevent the particulate material forming the intermediate layer from escaping from the longitudinal edges of the structure. The structure therefore can be produced separately and stored as it is, for example, as a continuous strip rolled in the form of a roll 11, shown in FIG. 2, which can subsequently be fed to the production line for disposable absorbent articles, for example sanitary napkins, where individual layered absorbent structures 12 are manufactured from the continuous, web-like, absorbent structure 14 in order to be incorporated as absorbent elements in the absorbent articles.

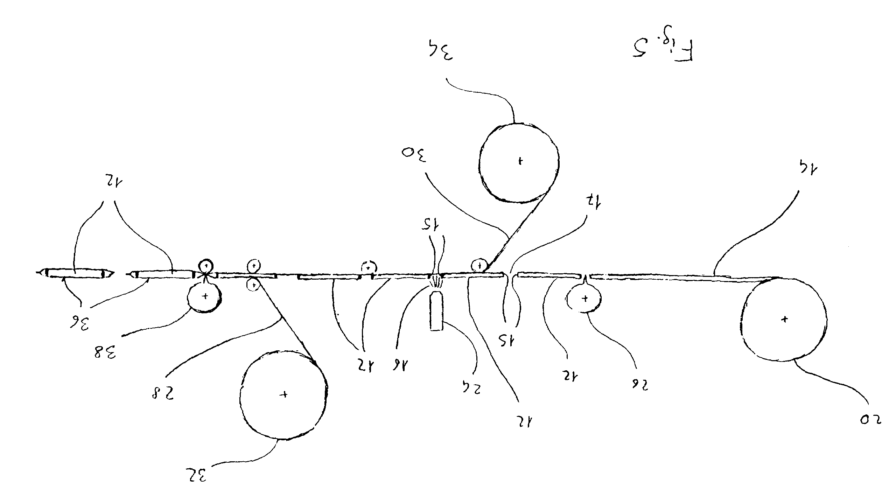

As illustrated in Figure 3, individual layered absorbent structures 12 of the desired length can be cut from a continuous, web-like, layered absorbent structure 14 of the type described above by means of cuts made along designated cutting lines 18 situated at predetermined intervals along the continuous structure 14 and indicated with a dashed line; as shown in Figure 3, the cuts are made in a direction transverse to the direction of feeding of the continuous structure 14 to the production line, which is indicated by an arrow and corresponds to the longitudinal direction of the continuous structure 14. In the embodiment illustrated in FIG. 3 the direction of the cut is perpendicular to the direction of feeding of the continuous structure 14, but non rectilinear cuts are also possible, for example curved cuts that provide each individual layered absorbent structure 12 with curved, usually convex, cut end edges. In the shown embodiment, cuts provide each individual layered absorbent structure 12 with an end surface 15 at each cut end edge. Individual layered absorbent structures 12, as well as the continuous web-like layered absorbent structure 14 from which they are achieved also have two major flat surfaces S substantially parallel to each other.

After the cutting step the individual layered absorbent structures 12 are spaced out from each other in longitudinal direction in order to provide a space 17 between the opposite end surfaces of each pair of consecutive individual layered absorbent structures 12.

An adhesive composition 16, not shown in FIG. 3 for clarity, is then applied to at least each end surface 15. The adhesive composition 16 provides joining between the containing layers 1 and 2 of each individual layered absorbent structure 12, and preferably provides the structure 12 with a seal at each end surface 15 therefore preventing the particulate material 6 and 9 from escaping from the cut edges of the individual layered absorbent structure 12. Loss or spillage of particulate material are therefore avoided.

FIG. 4 shows in greater detail the result of the application of the adhesive composition 16 to one of the end surfaces 15 of the layered absorbent structure 12 illustrated in FIG. 2. The adhesive composition 16 forms a substantially continuous layer that coats the end surface 15, therefore joining together the two fibrous layers 1 and 2 and preventing the particulate material 6 and 9 from escaping between them. Preferably the adhesive composition effectively provides the end surface 15 with a seal. As illustrated in FIG. 3 the adhesive composition 16 can also be applied, at least partially, to a narrow portion of one or both of the major flat surfaces S of the individual layered absorbent structure 12 that are coterminous with the respective end surfaces 15, in order to achieve a better sealing action. It is preferable however that the area of the major flat surfaces S of the individual layered absorbent structures 12 which is interested by the application of the adhesive composition 16 is kept to a minimum in order to avoid possible interactions with the absorption characteristics and with the softness of the individual layered absorbent structures 12.

It is not intended in the scope of the present invention that the adhesive composition 16 must totally entrap the particulate material at each end surface 15 of an individual layered structure 12, but rather that the adhesive composition 16 constitutes a joining means for the containing layers where the cut is made, entrapping a substantial percentage of the particulate material contained between them and therefore reducing the loss or spillage of particulate material. A minor loss of particulate material, e.g. through parts of the end surfaces where the adhesive composition is not applied, is within the scope of the present invention.

The adhesive composition 16 can be applied to the end surfaces 15 of the individual layered structures 12 in any suitable means known in the art, e.g. by spiral application, slot coating, spraying, spiral spraying, curtain coating, control coating and printing; preferably, the adhesive composition is applied by curtain coating since by this technique it is possible to direct with a better accuracy the adhesive composition where it is desired to apply it. Moreover, a curtain coater is capable of applying a layer of adhesive that is substantially continuous and has a uniform thickness which depends on the preferred amount of application.

Any suitable adhesive or glue, e.g. those known for the manufacture of disposable absorbent articles, can be used, such as for example water based or solvent based adhesives, or hot melt adhesives; preferably a hot melt adhesive can be used.

The adhesive composition can be applied in an amount between 15 g/m² and 60 g/m², preferably between 20 g/m² and 30 g/m².

In an example of the present invention a continuous, web-like, layered absorbent structure 14 of the type described previously and e.g. wound in a roll as that illustrated in FIG. 2 is fed to a production line of a sanitary napkin, said structure having a width of 70 mm and an overall thickness of 1.5 mm, and comprising:
• a first layer formed from a resin bonded air laid web of cellulose fibres 60 g/m²;
• an intermediate layer formed by a mixture of 63 g/m² of particles of absorbent gelling material, 61 g/m² of particles of zeolite, 87 g/m² of particles of silica, and 38 g/m² of particles of polyethylene;
• a second layer formed from a resin-bonded air laid web of cellulose fibres 60 g/m².

The two continuous lines of adhesive comprise two lines of hot-melt adhesive approximately 2 mm in width.

The continuous, web-like, layered absorbent structure 14 is cut transversely to the direction of feeding along each designated cutting line 18. Individual layered absorbent structures 12 that are 207 mm long are therefore provided being intended to be subsequently incorporated as absorbent elements in absorbent articles, e.g. sanitary napkins. Each individual layered absorbent structure 12 is provided with an end surface 15 at each edge where it is cut.

A hot melt adhesive composition 16 is applied in an amount of about 25 g/m² to both end surfaces 15 of each individual layered absorbent structure 12 and to a portion, that extends about 10 mm in Iongitudinal direction, of one of the major flat surfaces S of the individual structure 12 which is coterminous with each respective end surface 15, as can be easily recognized in FIG. 4, in order to join the two containing fibrous layers, and to provide a seal in said end surfaces 15.

Figure 5 is a simplified diagram of a process for producing a disposable absorbent article, e.g. a sanitary napkin, that uses as absorbent element an individual layered absorbent structure 12 obtainable from a continuous, web-like, layered absorbent structure 14 fed as a semi-finished product.

The reel 20 supplies the continuous, web-like, layered absorbent structure 14 to the production line. The continuous structure 14 is cut into individual layered absorbent structures 12 at the cutting station 26, and the individual structures 12 are spaced out from each other in the direction of the production line in order to provide spaces 17 between the opposite end surfaces 15 of each pair of consecutive individual layered absorbent structures 12.

The amount of said spaces 17 can be varied according to the process conditions, as can be readily determined by the skilled man, for example narrower spaces 17 will be provided for thinner individual sructures 12, while wider spaces 17 are needed for thicker individual structures 12, in order to allow a more effective subsequent application of the adhesive composition 16 to the end surfaces 15.

A fluid impervious backsheet 30 is then supplied to the production line from reel 34, and the individual layered absorbent structures 12 are associated to the backsheet 30 by known means, e.g. by adhesive means, not shown in the drawing for clarity.

The adhesive composition 16 is then applied by a curtain coater 24 to each pair of opposite end surfaces 15 belonging to consecutive individual structures 12. The application involves the end surfaces 15 and a narrow portion of the major flat surface S, facing the coater 24, of the individual layered structure 12 that is coterminous with each respective end surface 15. The application of the adhesive composition 16 further involves the backsheet 30 associated to the individual layered absorbent structures 12, in correspondence of each space 17 comprised between two consecutive individual layered absorbent structures 12. Therefore the adhesive composition 16 performs the further action of joining the individual layered absorbent structures 12 to the associated backsheet 30 at both end edges also, which correspond to the end surfaces 15.

After the application of the adhesive composition the backsheet 30 with the individual layered absorbent structures 12 associated and joined thereon are fed to an assembly station where a fluid pervious topsheet 28 is supplied from reel 32. The topsheet 28 and the backsheet 30 incorporate the individual structures 12 therebetween, and are finally joined together and cut along a perimeter at a cutting and sealing station 38 to form sanitary napkins 36 each comprising an individual structure 12.

According to an alternate embodiment of the process of the present invention the adhesive composition 16 can also be applied first directly to selected portions of a substrate layer, e.g. a backsheet, or a topsheet, were the end edges of the individual layered absorbent structures 12 are intended to be positioned, when the individual layered absorbent structures are subsequently associated to said substrate layer. The application of the adhesive composition onto the substrate must be such that it at least partially interests the end surfaces 15 of each pair of consecutive individual layered absorbent structures 12, after they have been associated to the substrate in correspondence of the respective selected portion where the adhesive composition has been applied, in order to achieve the joining in at least part of the end surfaces between the containing layers.

Although the containing layers of the layered structures described so far all have the same width which is constant along the length of the structure, further embodiments are also possible in which the containing layers have different widths, or, also, in which the width of the layered structure can vary along the length of the structure itself, in order to provide, for example, a shaped absorbent element for a disposable absorbent article, e.g. an hourglass shaped one, which can be manufactured from a continuous layered structure having a width varying along the longitudinal direction.

While the process of the present invention has been described in association with a continuous, web-like, layered structure having three distinct layers, it can be also applicable to different continuous layered structures, e.g. to structures having a different number of superimposed layers, or, alternatively, to another known type of C-folded absorbent laminate consisting of a single fibrous layer folded twice on itself and sealed by means of adhesive along the overlapping longitudinal side margins; the particulate material, e.g. absorbent gelling material and odour control material, is comprised therebetween. The particulate material and the superimposed portions of the fibrous layer are bonded together by means of sprayed adhesive. This type of structure is typically made on the production line of a sanitary napkin, rather than being produced as a semi-finished product to be subsequently fed to the production line. It does not have loss or spillage of particulate material along the longitudinal edges, but still has this problem when it is cut in transverse direction in the production line in order to form the individual layered structures that are to be incorporated in the sanitary products.

In an alternative embodiment of the present invention the process can also be used to produce individual layered structures by cutting a larger continuous layered structure along both longitudinal and transverse cutting lines and therefore providing the individual layered structures with end surfaces between the longitudinal and transverse cuts. The adhesive composition can be subsequently applied to all end surfaces of each individual layered structure, i.e. along the entire cut, therefore providing each individual layered structure with joining between the containing layers both in transverse and in longitudinal directions.

## Claims

1. A process for manufacturing individual layered structures comprising at least two containing layers, said process comprising the steps of:
a) providing a continuous web-like layered structure, said continuous, web-like layered structure having designated cutting lines and comprising a particulate material between said containing layers;
b) cutting said continuous web-like layered structure along said designated cutting lines to provide said individual layered structures having end surfaces between cuts;
c) spacing out said individual layered structures from one another so as to provide spaces between said end surfaces;
said process being characterized in that it comprises the further step of:
d) applying an adhesive composition to at least part of said end surfaces, so that said adhesive composition provides joining in said at least part of said end surfaces between said containing layers.

2. A process according to claim 1, characterized in that said adhesive composition comprises a hot melt adhesive.

3. A process according to any preceding claim, characterized in that said adhesive composition provides a seal in said at least part of said end surfaces where it is applied.

4. A process according to any preceding claim, characterized in that said containing layers are fibrous layers, preferably absorbent fibrous layers.

5. A process according to any preceding claim, characterized in that step a) is done by feeding said continuous web-like layered structure along a given direction, said continuous web-like layered structure having said designated cutting lines at predetermined intervals transversely with respect to said given direction, over the entire width of said continuous web-like layered structure.

6. A process according to claim 5, characterized in that said adhesive composition is applied to at least said end surfaces.

7. A process according to any preceding claim, characterized in that said adhesive composition is applied in an amount between 15 g/m² and 60 g/m², preferably between 20 g/m² and 30 g/m².

8. A disposable absorbent article comprising an absorbent element comprising an individual layered structure as obtained by a process according to anyone of claims 1 to 7.
